Europäisches Patentamt

European Patent Office

Office européen des brevets

(11) Publication number: **0 433 452 A1**

(12) **EUROPEAN PATENT APPLICATION**
published in accordance with Art.
158(3) EPC

(21) Application number: 90904427.3

(22) Date of filing: 08.03.90

(86) International application number:
PCT/JP90/00304

(87) International publication number:
WO 90/10708 (20.09.90 90/22)

(51) Int. Cl.⁵: **C12P 21/08, C12N 5/20,**
G01N 33/577, A61K 39/395,
//C12N15/06,C12R1:91

(30) Priority: 09.03.89 JP 55018/89

(43) Date of publication of application:
26.06.91 Bulletin 91/26

(84) Designated Contracting States:
AT BE CH DE DK ES FR GB IT LI LU NL SE

(71) Applicant: SENDAI INSTITUTE OF
MICROBIOLOGY
5-12, Hayama-cho Sendai-shi
Miyagi(JP)

(72) Inventor: MIZUGAKI, Michinao 1-7-33, Kunimi
Aoba-ku
Sendai-shi
Miyagi 981(JP)
Inventor: ITOH, Kunihiko 13-4, Aza Genbei
Nishi
Koriyama Taihaku-ku
Sendai-shi Miyagi 982(JP)
Inventor: ISHIDA, Nakao 1-5-40, Tsunogoro
Aoba-ku
Sendai-shi
Miyagi 980(JP)

(74) Representative: Kraus, Walter, Dr. et al
Patentanwälte Kraus, Weisert & Partner
Thomas-Wimmer-Ring 15
W-8000 München 22(DE)

(54) MONOCLONAL ANTIBODY, AND ASSAY METHOD, REAGENT KIT, SEARCH METHOD AND DRUG MISSILE USING SAID ANTIBODY.

(57) The invention relates to a monoclonal antibody which can recognize lower alkyl cytidines, hybridoma which produces the monoclonal antibody, and a method of assaying lower alkyl cytidines in a specimen such as a bodily fluid, a reagent kit, an immunohistochemical search method and a drug missile, each utilizing the monoclonal antibody. The invention facilitates diagnosis and treatment of a cancer.

## MONOCLONAL ANTIBODIES, ASSAY METHOD, REAGENT KIT, SEARCHING METHOD AND DRUG MISSILES USING THEM

### Technical Field

This invention concerns monoclonal antibodies, in particular monoclonal antibodies against 5-methylcytidine and other modified nucleosides, which are elevated in the body fluids of cancer patients.

This invention also concerns hybridomas which produce such monoclonal antibodies, an immunoassay method, reagent kit, immunohistochemical searching method and drug missiles using the monoclonal antibodies.

### Background Art

In the cancer diagnosis and postoperational monitoring, it is very important to assay the cancer-related substances, or the so-called tumor markers, which are elevated in the patients' serum or urine. The tumor markers currently assayed include carcinoembryonic antigen (CEA), immunosuppressive acidic protein (IAP), $\alpha$-fetoprotein (AFP) specific to the liver cancer, and CA19-9 which is a sugar chain antigen specific to the pancreatic cancer. Meanwhile, it has long been known that a variety of modified nucleosides are elevated in the urine of cancer patients.

The present inventors formerly assayed the urines and sera of cancer patients and tumor-bearing animals by a conventional technique of high performance liquid chromatography (HPLC) in order to determine the usefulness of modified nucleosides as a tumor marker. As a result, it was found that 5-methylcytidine was abnormally elevated in cancer patients and tumor-bearing animals.

In this way the usefulness of this compound as a tumor marker has been proven, but the HPLC analysis requires a complicated sample pretreatment which takes a long time, especially, when assaying many samples. In addition, it has been impossible to study the subcellular localization of 5-methylcytidine and the variation of its amount on the cellular level by an immunohistochemical technique.

The present inventors have made extensive studies for the development of a simplified assay method of 5-methylcytidine and have found that use of a newly prepared monoclonal antibodies against 5-methylcytidine makes it possible, without any pretreatment of samples, to assay many samples at the same time by an modified enzyme immunoassay technique, thus providing a faster and simpler assay. This has led us to the completion of this invention.

### Disclosure of Invention

This invention concerns monoclonal antibodies which can recognize lower alkyl-cytidines and hybridomas which produce said monoclonal antibodies.

This invention also concerns an immunological technique for estimating lower alkyl-cytidines in a sample, in particular, an assay method of using monoclonal antibodies which can recognize lower alkyl-cytidines; a reagent kit for immunologically estimating lower alkyl-cytidines in a sample, in particular, a reagent kit including monoclonal antibodies which can recognize lower alkyl-cytidines and conjugates of lower alkyl-cytidines and carrier protein-II; and a method of immunohistochemically searching modified nucleosides which are accumulated in tumor tissues as a consequence of abnormally elevated metabolism of nucleic acids, in particular, a method wherein monoclonal antibodies which can recognize the modified nucleosides are immunologically bound to a part of the tissues.

This invention, furthermore, concerns drug missiles with the characteristic feature of using, as a carrier for anti-cancer or carcinostatic drugs, monoclonal antibodies which can recognize the modified nucleosides.

### Brief Explanation of the Drawings

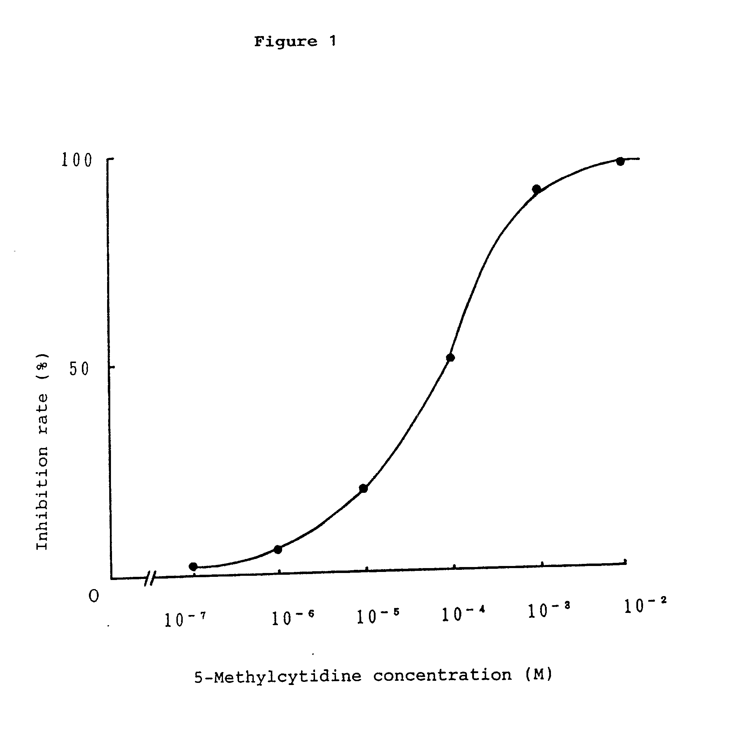

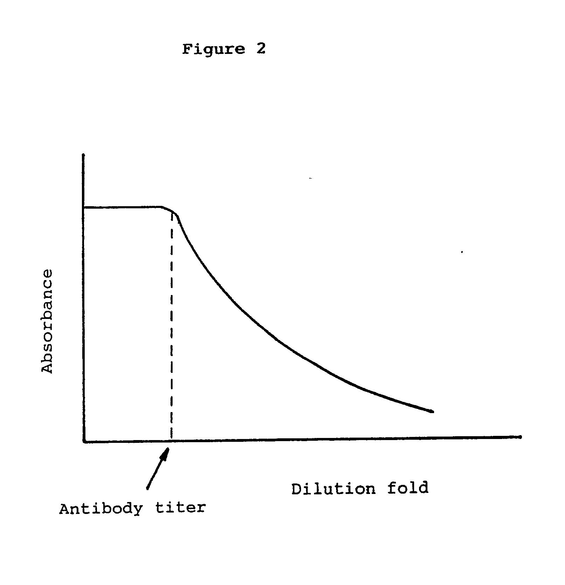

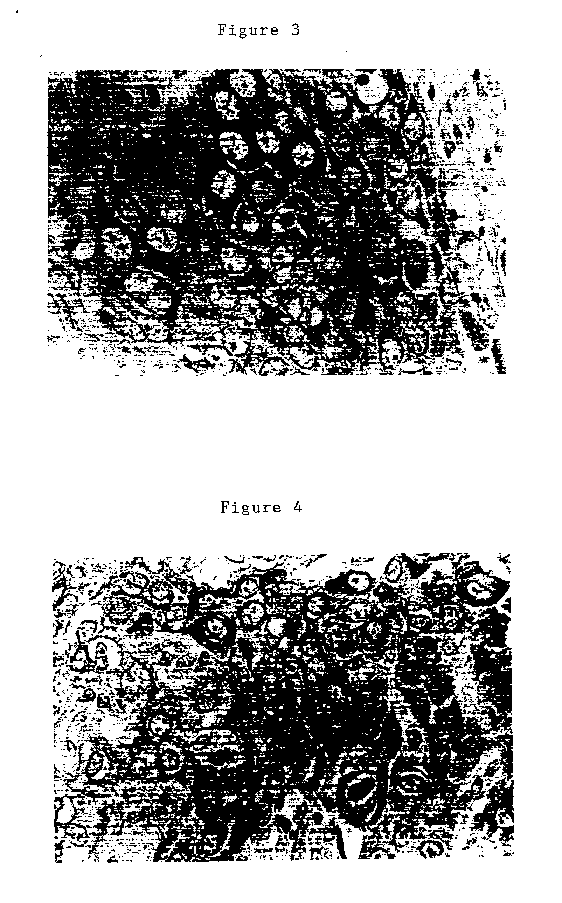

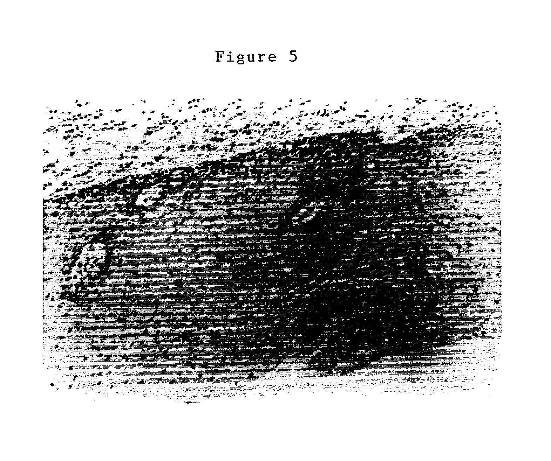

Figure 1 shows a detection characteristics of 5-methylcytidine detected by MCT-3, one of the monoclonal antibodies according to this invention. Figure 2 is a diagram showing the relation between the dilution fold and absorbance in obtaining the antibody titer of the monoclonal antibodies according to this invention. Figure 3 gives the results of immunological staining of tissues of esophagus cancer with an anti-1-methyladenosine monoclonal antibody while Figure 4 shows the results of immunostaining of the same tissues with anti-pseudouridine monoclonal antibody. Figure 5 is a photograph showing the result of immunostaining of a normal esophagus tissue with anti-1-methyladenosine monoclonal antibody.

2

Best Mode of the Invention

The monoclonal antibodies according to this invention are those which can recognize specifically lower alkyl-cytidines. As the lower alkyl-cytidines may be mentioned the cytidines in which hydrogen atom(s) is substituted by straight or branched chain lower alkyl groups having 1 - 5 carbon atoms such as methyl, ethyl, propyl, isopropyl, butyl, isobutyl, pentyl and isopentyl groups. Among these lower alkyl groups, methyl is the most preferred. Favorable positions for the introduction of the lower alkyl groups are 3-, 5- and 6- positions in the pyrimidine nucleus constituting cytidine, among which 5-position is particularly preferred.

Among the monoclonal antibodies of this invention, the most preferable one is the antibody which recognizes 5-methylcytidine in view of the assay precision of the modified nucleosides as a tumor marker and safety.

As the class for such monoclonal antibodies, IgG is favorable, and $IgG_1$ and $IgG_{2a}$ are particularly preferred. The monoclonal antibodies of the class $IgG_1$ show high reaction specificity in the recognition of 5-methylcytidine and have high antibody titers, thus practically advantageous.

The monoclonal antibodies of this invention can recognize lower alkyl-cytidines, and it is desirable that they can recognize the alkyl-cytidines present in animals' urine or in body fluids such as blood and lymph. In particular, it is practically preferable that the antibodies can recognize lower alkyl-cytidines in human body fluids.

As a favorable example of producing the monoclonal antibodies which can recognize lower alkyl-cytidines of this invention may be given the following method, where a monoclonal antibody recognizing 5-methylcytidine is prepared:

First, an animal is immunized with 5-methylcytidine, and spleen cells are taken out of the animal. In this procedure, since 5-methylcytidine itself cannot be an immunogen, it is conjugated with an appropriate carrier protein-I and used as an immunogen.

5-Methylcytidine to be used in this invention is any one of those obtainable from the urine of cancer patients by isolation and purification, obtainable by synthesis or commercially available authentic preparations. As the carrier protein-I, such proteins which are used for the purpose of making the immunocompetent cells recognize the substances active as haptens are used, which include keyhole limpet hemocyanin, bovine serum albumin, human serum albumin and eggwhite albumin. An examplary method of conjugating 5-methylcytidine and carrier protein-I is oxidizing the sugar moiety of a nucleic acid base with periodic acid and then making it conjugate with the carrier protein-I.

Animals to be immunized with the conjugate of 5-methylcytidine and carrier protein-I include mice and rats, among which mice are practically advisable.

Then, spleen cells of the immunized animal are fused with myeloma cells to obtain a hybridoma. The cell fusion may be performed by either one of the known methods using polyethyleneglycol (Marc Shulman, C.D. Wide & G. Köhler, Nature 276, 269 - 270 (1978)), a method of fusion utilizing Sendai virus (G. Köhler & C. Milstein, Nature 256, 495 - 497 (1975), a method according to Eur. J. Immunol. 6, 511 - 519 (1976)), or a method utilizing electric pulses (J. Vienken & U. Zimmermann, FEBS Letters 137, 11 - 13 (1982)). In the screening of hybridomas, it is necessary to remove the hybridomas that produce antibodies against the carrier protein. For this purpose it is desired to do screening of the antibody-producing hybridomas, for example, by using, as an antigen, a substance obtained by conjugating a protein derived from a species different from that used for the immunization with 5-methylcytidine.

The spleen cells and myeloma cells used for the production of the monoclonal antibodies of this invention via hybridomas are favorably those derived from animals of the same species. Practically, mouse spleen cells and myeloma cells derived from a mouse are particularly preferred.

In the next place, the selected hybridomas or the transformed cells are cultured to make them form desired specific monoclonal antibodies. The hybridomas or transformed cells selected by cloning and produce antibodies which recognize lower alkyl-cytidines may be preserved frozen. It is also possible to culture them in a large scale by an adequate technique. From the culture supernatant, monoclonal antibodies that are conjugated specifically with lower alkyl-cytidines can be obtained. It is also possible to prepare desired antibodies from ascites or serum of an animal into which these cells are transplanted for the formation of tumors. Purification of the monoclonal antibodies of this invention is performed by affinity chromatography or other techniques.

This invention also includes hybridomas which are obtained by the fusion of spleen cells capable of producing antibodies that can recognize lower alkyl-cytidines and that are prepared by using a conjugate of lower alkyl-cytidines and carrier protein-I as an immunogen and myeloma cells. As a method of obtaining such hybridomas, the method of preparing hybridomas described above in the production of monoclonal antibodies is favorably employed.

The inventors of this invention have completed the assay method of this invention, where lower alkyl-cytidines, particularly favorably 5-methylcytidine, a marker of progressive cancer in the patients' urine, serum and other body fluids can be assayed simply and in high precision by using these monoclonal antibodies that can recognize lower alkyl-cytidines.

Thus the assay method of this invention has a characteristic feature of using monoclonal antibodies that recognize lower alkyl-cytidines among the assay methods of lower alkyl-cytidines in test samples by the immunological technique. Favorable test samples (designated simply as samples hereafter) in this assay method are intact or processed body fluids of animals, particularly of human being, and the use of intact body fluids makes the process simpler and more advisable.

In the assay method of this invention, it is preferable to use monoclonal antibodies that recognize lower alkyl-cytidines as fixed to a carrier. The fixation can be performed by a known technique, and as the carrier such solid ones as balls, beads, gear and microplates made of polystyrene, polyethylene, polyacrylate, teflon, polyacetate and other materials may be favorably utilized.

The method and procedures of labeling the monolonal antibodies and the method and means of its detection are not limited at all, and known methods and procedures may be employed, for example, those using the anti-immunoglobulin antibody or staphylococcal protein A which are labeled with a radioactive substance, an enzyme or a fluorescent substance. As the labeling substances, horse-radish peroxidase, $\beta$-D-galactosidase, alkaline phosphatase, and other enzymes may ordinarily be used in the methods using an enzyme (enzyme immunoassay, EIA); $^{125}$I, $^3$H and other isotopes in those using a radioactive substance (radio-immunoassay, RIA); and fluorescein isothiocyanate and other compounds in those using a fluorescent substance (fluoroimmunoassay, FIA), but so far as the activity of the labeling substance is readily measurable, other substances may also be utilized.

When the labeling substance is an enzyme, a substrate is used for assaying its activity. Examples of the substrates for horseradish peroxidase are 2,2'-azinodi-[3-ethylbenzthiazoline-sulfonic acid] diammonium salt (ABTS)-$H_2O_2$, 5-aminosalicylic acid-$H_2O_2$, O-phenylenediamine-$H_2O_2$ and 4-aminoantipyrine-$H_2O_2$, and examples of the substrate for $\beta$-D-galactosidase are fluorescein-di-($\beta$-D-galactopyranoside) and O-nitrophenyl-$\beta$-D-galactopyranoside. For assaying the enzyme activities, known reagents of solvents, washing agents and reagents for stopping reaction are used other than the above reagents. A method utilizing the polarized light of the antigen-antibody complex may also be used.

For the practice of the immunoassay method of this inventon, an example of determining 5-methylcytidine is given:

To a 96-well microplate is added a conjugate of 5-methylcytidine and bovine serum albumin (BSA) in a quantity of 1 $\mu$g/well and the plate is kept at 4°C for 12-24 hours. Then 100 $\mu\ell$ each of phosphate-buffered saline (PBS) containing 1% BSA is added to the well to prevent unspecific adsorption of antibody and other proteins. A sample (urine, blood or other materials) is added to the wells in an amount of 50 $\mu\ell$ each. Then, the equal volume of antibody solution was added to each well. After through agitation, the plate is left at 4°C for 1 hour. The wells are washed thoroughly with PBS and added with 100 $\mu\ell$ each of the 3000-fold diluted solution of alkaline phosphatese-labeled, goat anti-mouse IgG antibody. After reaction is run at 4°C for 45 minutes, the wells are washed with PBS and moisture is removed. 100 $\mu\ell$ each of the substrate solution (1 M diethanolamine buffer (pH 9.8) containing p-nitrophenyl phosphate in a concentration of 1 mg/ml) is added thereto, and the mixture is allowed to react at 37°C for 30 minutes. By measuring absorbance at 405 nm in each well by an EIA reader, the amount of 5-methylcytidine present in the sample is determined.

This invention also includes in the scope a reagent kit with the characteristic feature of using a monoclonal antibody which can recognize lower alkyl-cytidines and a conjugate of lower alkyl-cytidines and carrier protein-II, among kits for immunologically assaying lower alkyl-cytidines in test samples.

The monoclonal antibodies here mentioned have the characteristic features as described heretofore. It is necessary that protein-II which conjugates with lower alkyl-cytidines be selected from the protein groups similar to those of the above-mentioned carrier protein-I, and that the antigenicity of the conjugate be different from that of carrier protein-I.

The reagent kit of this invention may also contain, other than the monoclonal antibodies and the conjugates, a labeling agent and a reagent for detection of the labeled antibodies, if desired.

This invention furthermore includes a method of immunohistochemically detecting the presence of modified nucleosides which have been accumulated in tumor tissues as a result of abnormal elevation of metabolism of nucleic acids, characterized in that monoclonal antibodies capable of recognizing modified nucleosides are bound to a part of tissues by an immunoreaction.

The tissues in such an immunohistochemical searching or detecting method mean cells or tissue sections of animals, in particular of human being which are practically favorable.

As the monoclonal antibodies to be used in the searching method of this invention, anti-pseudouridine monoclonal antibody (Japanese Patent Kokai Publication No. 299765 (1987), No. 222699 (1988)), anti-1-methyladenosine monoclonal antibody (Japanese Patent Kokai Publication No. 299766 (1987)) and other monoclonal antibodies against cancer-related, modified nucleosides may also be mentioned beside the above antibodies against lower alkyl-cytidines.

In more concrete sense, the searching method of this invention has the characteristic feature of causing an immunoreaction by allowing the tissues to react with the monoclonal antibodies capable of recognizing modified nucleosides, thereby to have the monoclonal antibodies bound to the tissues at the site where the nucleosides are present. Thereafter, the tissues are treated, for example, with a biotinylated antibody that can recognize such monoclonal antibodies and subsequently with enzyme-labeled avidin for revealing the site of localization of said modified nucleosides in the tissues by staining or other labeling technique so as to make searching the site of progressive cancers possible.

The immunohistochemical searching method described below is an example of an embodiment of the searching method of this invention. In the method of this invention, cells or tissue sections are fixed by using acetone, formalin, paraformaldehyde or other chemicals and treated with PBS containing 1% BSA to prevent nonspecific adsorption of proteins. Then the monoclonal antibodies are allowed to react and left at room temperature for 30 minutes. After thorough washing with PBS, the biotinylated anti-mouse IgG antibody is allowed to react and left at room temperature for 30 minutes. After thorough washing with PBS, avidin-biotinylated peroxidase complex (ABC) is allowed to react and left at room temperature for 30 minutes and washed thoroughly with PBS. In the next place, the substrate solution (PBS (pH 7.4) containing 0.5 mg/ml of diamino-benzidine and 0.01% hydrogen peroxide) is added for staining. Subcellular localization of desired modified nucleosides is looked for under an optical microscope.

In this invention is also included drug missiles with the characteristic feature of using, as the carrier of anticancer or carcinostatic drugs, monoclonal antibodies that can recognize modified nucleosides accumulated as a result of abnormal elevation of metabolism of nucleic acids in the tumor tissues.

Such anticancer or carcinostatic drugs may be, for example, such alkylating agents as chlorambutyl, ACNU, CCNU and cisplatin; such metabolic antagonists as MTX, 5FU, FUDR and ARA-C; such vinca alkaloids as vinblastine and vincristine; and such antibiotics as MMC, Adriamycin and Daunomycin, and any other agents may be utilized so far as their monoclonal antibodies can be used as a carrier, which should not be construed as limiting this invention thereto.

Any form of application is possible for using the monoclonal antibodies as a carrier, including a therapeutic method utilizing the antibodies as a carrier of radioisotopes. The radioisotopes may be $\alpha$-emitters such as $^{212}Bi$ and $^{211}At$ and $\beta$-emitters such as $^{131}I$ and $^{90}Y$.

By using the drug missiles of this invention, the anticancer and carcinostatic drugs can be brought to the affected site efficiently, which leads to an enhanced therapeutic effect and lower adverse side effects.

This invention also includes monoclonal antibodies suitably usable in the imaging diagnosis, which can recognize lower alkyl-cytidines and which are labeled by radioactive, paramagnetic or fluorescent substances.

The radioactive substances here mentioned are, for example, $^{125}I$, $^{111}In$ and $^{99m}Tc$ and an example of the paramagnetic substances is gadolinium (Gd). Any substances may be utilized, so far as it serves as a label for the monoclonal antibodies of this invention and when the labeled antibodies are introduced into animal bodies, particularly favorably into human bodies, they are brought to the sites where lower alkyl-cytidines are localized for labeling the sites, thus serving for imaging diagnosis with the aid of radioactive rays and ultrasonic wave.

By using such monoclonal antibodies of this invention for the imaging diagnosis, the presence of progressive cancers which are accompanied with the formation of lower alkyl-cytidines in the body, their localization and their progression speed can be easily diagnosized.

The antibody titer used in this invention which indicates the characteristics of the monoclonal antibodies is explained taking 5-methylcytidine as an example. A conjugate of 5-methylcytidine and bovine serum albumin (BSA) is made to be adsorbed previously to a 96-well microplate, and to the wells are added serially 2-fold diluted solutions of the supernatant of a culture medium of hybridomas which produce the monoclonal antibody of this invention to allow an immunoreaction to proceed, and then absorbance of each well is estimated. The titer means the maximum dilution fold that can maintain the maximum absorbance. A diagrammatic representation of the pattern of the relation between such a dilution fold and absorbance, from which the antibody titer is obtained is given in Figure 2.

Examples

5

Below are given examples of the embodiments of this invention, but they do not limit the scope of this invention.

Example 1

(1) Preparation of Immunogen:

A conjugate of 5-methylcytidine, purchased from Sigma, and keyhole limpet hemocyanin, a carrier protein-I, was prepared by the method of Erlanger and Bieser (Proc. Natl. Acad. Sci., 52, 68 (1964)). Namely, 5-methylcytidine was oxidized by periodic acid, and after decomposing excess periodic acid with ethyleneglycol, it was allowed to react with hemocyanin under alkaline condition (pH 9 - 9.5). Then the reaction mixture was reduced with sodium borohydride to form a stable compound. The reaction mixture was dialyzed overnight in PBS (pH 7.4) to remove unreacted 5-methylcytidine, and after lyophilization it was stored in a freezer at -20° C.

(2) Preparation of Monoclonal Antibody:
(i) The conjugate of 5-methylcytidine and hemocyanin, which was obtained in (1), was admixed with an same amount of the Freund's complete adjuvant to make an emulsion, and administered intraperitoneally to BALB/c mice in a dose of 50 $\mu$g per head. In the second and following administrations, the emulsion made with incomplete adjuvant was employed in 50 $\mu$g per mouse, being given intraperitoneally twice with a 14 day interval. As a final immunization, 0.2 ml of a 100 $\mu$g/ml solution of the conjugate of 5-methylcytidine and hemocyanin was intravenously injected.
(ii) Three days after the final immunization, the spleen cells isolated from hyperimmunized mouse and the myeloma cells strain Sp2/O-Ag14, derived from BALB/c mouse were fused by using polyethyleneglycol 4000. The cells were placed in a 96-well microplate in a volume of 100 $\mu$l/well, and after 24 hours, half a volume of the culture medium was replaced by a HAT medium and this medium replacement was repeated every third day. After 7 - 10 days, hybridomas resistant to HAT were found to grow. At this time the medium was changed to a HT medium and after 10 days culturing, it was changed to a hybridoma-growing medium.
(iii) Screening of the antibody-producing cells was performed by the two-antibody ELISA method by using a conjugate of 5-methycytidine and bovine serum albumin (BSA) as an antigen. By this method, abolishing the most worried-about hybridomas which produce the antibodies against the carrier protein was successfully achieved. Then by examining inhibition by 5-methylcytidine, the hybridomas producing the monoclonal antibodies specifically reactive with 5-methylcytidine were selected. The hybridomas here selected were cloned by the limiting dilution method to establish a hybridoma clone which produces monoclonal antibody. Hybridoma MCT-3, one of such clones, have been deposited as FERM BP-2788 in the Fermentation Research Institute, Agency of Industrial Science and Technology.
(3) Properties of the Monoclonal Antibodies:

The monoclonal antibodies thus prepared were classified into 3 groups based on their reaction specificities:
(i) The monoclonal antibodies that react very strongly with 5-methylcytidine (MCT-3 and four other clones), (ii) those showing lower reactivity with 5-methylcytidine than the monoclonal antibodies in (i) but do not react with other nucleosides (MCT-6 and one other clone), and (iii) the monoclonal antibodies that react with 5-methylcytidine, 5-methylcytosine and furthermore thymidine and cytidine (MCT-8 and one other clone). Regarding the monoclonal antibodies showing the highest reactivity in these groups, namely MCT-3, MCT-6 and MCT-8, the antibody titers and reaction specificities are shown in Table 1.

Table 1

| Monoclonal antibody | | MCT-3 | MCT-6 | MCT-8 |
|---|---|---|---|---|
| Antibody class | | $IgG_1$ | $IgG_1$ | $IgG_2a$ |
| Antibody titer | | 64-fold | 16-fold | 32-fold |
| Reaction specificity* | 5-Methylcytidine | + + | + | + + + |
| | 5-Methylcytosine | − | − | + |
| | Cytidine | − | − | + |
| | Thymidine | − | − | + + |

*)   +++ : High reactivity

++ : Medium reactivity

+ : Low reactivity

− : No reactivity

Example 2

To a 96-well microplate which had been coated with 5-methylcytidine-BSA (0.1 μg/well) were added 50 μℓ each of $10^{-2}$, $10^{-3}$, $10^{-4}$, $10^{-5}$, $10^{-6}$ and $10^{-7}$ M solutions of 5-methylcytidine and then 30-fold diluted solution of MCT-3 monoclonal antibody to conduct a competitive reaction. The results demonstrated, as shown in Figure 1, that in proportion to the amount of 5-methylcytidine added, the binding of MCT-3 antibody and 5-methylcytidine-BSA is inhibited. When this inhibition curve was used as the calibration curve, the detection limit of 5-methylcytidine was 1 pmol, and therefore this assay system has shown to provide a highly sensitive assay method of minute amounts of 5-methylcytidine.

Example 3

By replacing the 5-methylcytidine solutions in Example 2 with urine of healthy donors and cancer patients, the similar competitive inhibition test was conducted. By using the calibration curve obtained in Example 2, 5-methylcytidine in the urine samples was assayed. The results are shown in Table 2.

Table 2
Assay of 5-Methylcytidine in Healthy Persons and
Cancer Patients by Using MCT-3

| Cases | 5-Methylcytidine (n mol/$\mu$mol creatinine) |
|---|---|
| Healthy person | 2.41 ± 0.03 |
| Healthy person | 3.25 ± 0.12 |
| Healthy person | 1.71 ± 0.02 |
| Hepatoma | 34.92 ± 0.89 |
| Gastric cancer | 35.36 ± 1.26 |
| Gastric cancer | 19.91 ± 0.56 |
| Gastric cancer | 17.38 ± 1.06 |
| Gastric cancer | 18.75 ± 1.39 |

Example 4

Following the procedures described below, immunohistological staining of esophageal cancer tissues with the use of anti-modified nucleoside monoclonal antibodies is performed.

(1) Esophageal cancer tissue (a preparation of operative isolation) was fixed with formalin and embedded with paraffin and sections of 3 - 5 $\mu$m size were prepared.

(2) The sections were placed on a slide glass and treated with normal horse serum to prevent nonspecific adsorption.

(3) Then the sections were treated by allowing to react with a monoclonal antibody (anti-1-methyladenosine antibody or anti-pseudouridine antibody, in a concentration of 5 $\mu$g/ml) for 1 hour.

(4) The slide glass was thoroughly washed with the buffer solution and treated with biotinylated anti-mouse IgG by the reaction at room temperature for 30 minutes.

(5) The slide glass was thoroughly washed with the buffer solution and treated with the horseradish peroxidase-avidin complex by the reaction at room temperature for 30 minutes.

(6) After the slide glass was thoroughly washed with the buffer solution, the substrate solution (phosphate buffer solution containing 0.5 mg/ml of diaminobenzidine and 0.01% hydrogen peroxide solution) was added for coloration.

(7) The nucleus was stained with a hematoxylin solution.

The results are shown in Figures 3 - 5. The results demonstrated that use of either anti-1-methyladenosine monoclonal antibody or anti-pseudouridine monoclonal antibody gave no staining with normal tissues but gave specific staining with cancerous tissues. In Figures 3 and 4 the parts in dark gray to black colors are the stained parts (reaction positive sites).

Industrial Applicability

The monoclonal antibodies of this invention have excellent characteristics of recognizing in high sensitivity lower alkyl-cytidines, in particular 5-methylcytidine. The hybridomas of this invention have such useful properties as producing the monoclonal antibodies having such excellent characteristics. By using the assay method and the reagent kit of this invention, lower alkyl-cytidines can be assayed easily without prior treatment of body fluids and other samples, and thus it is possible to do assaying of many samples simply and promptly for judging the presence of progressive cancer.

Also by the use of the searching method of this invention, detection of modified nucleosides which are accumulated as the result of abnormal elevation of metabolism of nucleic acids in tumor tissues can be performed easily. The monoclonal antibodies of this invention for imaging diagnosis make it possible to diagnose the cancers which are progressive in the body very simply, and the drug missiles provide anticancer and other drugs with higher therapeutic effects by bring them efficiently to the affected sites.

**Claims**

1. A monoclonal antibody which can recognize a lower alkyl-cytidine.

2. The monoclonal antibody of Claim 1, in which said lower alkyl-cytidine is a 5-lower alkyl-cytidine.

3. The monoclonal antibody of Claim 1, in which said lower alkyl-cytidine is 5-methylcytidine.

4. The monoclonal antibody of Claim 1, in which said lower alkyl-cytidine is contained in a biological sample.

5. The monoclonal antibody of Claim 1, which is produced by a hybridoma obtained by the fusion of spleen cells which have been prepared using a conjugate of a lower alkyl-cytidine and carrier protein-I as an immunogen and myeloma cells.

6. The monoclonal antibody of Claim 5, in which said spleen cells are mouse spleen cells and said myeloma cells are mouse-derived myeloma cells.

7. The monoclonal antibody of Claim 1, in which the class of said antibody is IgG.

8. The monoclonal antibody of Claim 7, in which said IgG is $IgG_1$.

9. The monoclonal antibody for use with the imaging diagnosis of Claim 1, said antibody being labeled with one or more substances selected from the group consisting of radioactive substances, paramagnetic substances and fluorescent substances.

10. A hybridoma obtained by the fusion of spleen cells capable of producing antibodies which can recognize a lower alkyl-cytidine, and are prepared by immunizing an animal with a conjugate of a lower alkyl-cytidine and carrier protein-I as an immunogen and myeloma cells.

11. An assay method for lower alkyl-cytidines in samples by an immunological technique, characterized by using a monoclonal antibody which can recognize lower alkyl-cytidines.

12. A reagent kit for immunologically assaying lower alkyl-cytidines in samples, characterized by using a monoclonal antibody which can recognize lower alkyl-cytidine and a conjugate of a lower alkyl-cytidine and carrier protein-II.

13. An immunohistochemical searching method for detecting the presence of a modified nucleoside which is accumulated in tumor tissues as a result of abnormal elevation of metabolism of nucleic acids characterized in that a monoclonal antibody which can recognize said modified nucleoside is bound to a part of tissue by immunoreaction.

14. The searching method of Claim 13, in which said modified nucleoside is a lower alkyl-cytidine.

15. The searching method of Claim 13, in which said modified nucleoside is a pseudouridine.

16. The searching method of Claim 13, in which said modified nucleoside is 1-methyladenosine.

17. A drug missile characterized in that a monoclonal antibody which can recognize a modified nucleoside accumulated in tumor tissues as a result of abnormal elevation of metabolism of nucleic acids is used as a carrier of anticancer or carcinostatic drugs.

18. The drug missile of Claim 17, in which said modified nucleoside is a lower alkyl-cytidine.

19. The drug missile of Claim 17, in which said modified nucleoside is pseudouridine.

20. The drug missile of Claim 17, in which said modified nucleoside is 1-methyladenosine.

Figure 1

5-Methylcytidine concentration (M)

Figure 2

Absorbance

Antibody titer

Dilution fold

Figure 3

Figure 4

Figure 5

# INTERNATIONAL SEARCH REPORT

## I. CLASSIFICATION OF SUBJECT MATTER (if several classification symbols apply, indicate all) ⁶

According to International Patent Classification (IPC) or to both National Classification and IPC

Int. Cl⁵     C12P21/08, C12N5/20, G01N33/577, A61K39/395//
           C12N15/06 (C12P21/08, C12R1:91)

## II. FIELDS SEARCHED

### Minimum Documentation Searched ⁷

| Classification System | Classification Symbols |
|---|---|
| IPC | C12P21/08, C12N5/20, G01N33/577, A61K39/395 |

Documentation Searched other than Minimum Documentation
to the Extent that such Documents are Included in the Fields Searched ⁸

Biological Abstracts Data Base (BIOSIS)
Chemical Abstracts Data Base (CA)

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| Y | Chemical Abstracts, Vol.95, No.7, 17 August 1981 (17. 08. 81), (Columbus, Ohio, U. S. A.), See page 514, abstract No.59731d, Z. Naturforsch., C: Biosci., 1981, 36c (5-6), 459-63 (Eng) | 1 - 12 |
| Y | Chemical Abstracts, Vol.97, No.9, 30 August 1982 (30. 08. 82), (Columbus, Ohio, U. S. A.), See page 481, abstract No.70596a, Z. Naturforsch., C: Biosci., 1982, 37c (5-6), 399-404 (Eng) | 1 - 12 |
| Y | Chemical Abstracts, Vol.103, No.19, 11 November 1985 (11. 11. 85), (Columbus, Ohio, U. S. A.), See page 555, abstract No.158797v, Eur. J. Biochem., 1985, 152 (1), 115-21 (Eng) | 1 - 12 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| May 31, 1990 (31. 05. 90) | June 18, 1990 (18. 06. 90) |

| International Searching Authority | Signature of Authorized Officer |
|---|---|
| Japanese Patent Office | |

Form PCT/ISA/210 (second sheet) (January 1985)

| | FURTHER INFORMATION CONTINUED FROM THE SECOND SHEET | |
|---|---|---|
| A | JP, A, 61-116660 (Jahn Rolland Holmgren and two others), 4 June 1986 (04. 06. 86) & EP, A2, 173663 & SE, A, 8404283 | 1 - 20 |

**V.☐ OBSERVATIONS WHERE CERTAIN CLAIMS WERE FOUND UNSEARCHABLE [1]**

This international search report has not been established in respect of certain claims under Article 17(2) (a) for the following reasons:

1.☐ Claim numbers          , because they relate to subject matter not required to be searched by this Authority, namely:

2.☐ Claim numbers          , because they relate to parts of the international application that do not comply with the prescribed requirements to such an extent that no meaningful international search can be carried out, specifically:

3.☐ Claim numbers ...          , because they are dependent claims and are not drafted in accordance with the second and third sentences of PCT Rule 6.4(a).

**VI.☐ OBSERVATIONS WHERE UNITY OF INVENTION IS LACKING [2]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees

Form PCT/ISA/210 (supplemental sheet (2)) (January 1985)

| | | |
|---|---|---|
| FURTHER INFORMATION CONTINUED FROM THE SECOND SHEET | | |
| Y | Nature, Vol.256, (1975),<br>G. Köhler and C. Milstein,<br>"Continuous cultures of fused cells<br>secreting antibody of predefined<br>specificity" p.495-7 | 1 - 12 |
| A | JP, A, 60-67431 (Mitsubishi Chemical<br>Industries Ltd.),<br>17 April 1985 (17. 04. 85),<br>(Family: none) | 1 - 20 |

**V.☐ OBSERVATIONS WHERE CERTAIN CLAIMS WERE FOUND UNSEARCHABLE** [1]

This international search report has not been established in respect of certain claims under Article 17(2) (a) for the following reasons:

1.☐ Claim numbers , because they relate to subject matter not required to be searched by this Authority, namely:

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest

☐ No protest accompanied the payment of additional search fees